# EUROPEAN PATENT APPLICATION

(11) **EP 0 923 923 A2**
(43) Date of publication of application: **23.06.1999**
(21) Application number: 98123805.8
(22) Date of filing: 15.12.1998
(51) Int. Cl.: A61H 35/00

(54) **Running-water treatment device for the human or animal body**

(30) Priority: 22.12.1997 IT MI972847
(71) Applicant: BADI FARM S.r.l., 21013 Gallarate (Varese) (IT)
(72) Inventor: Badi, Ferruccio, 21040 Albusciago (Varese) (IT)
(74) Representative: Giambrocono, Alfonso, Dr. Ing.

(57) **Abstract**

The running-water treatment device for the human or animal body comprises a bag which is deformable to allow it to adapt to that part of the body to be treated. The bag is fed with cold or tepid water and is provided with a multiplicity of holes through which the water emerges, to wet with a continuous flow of water that part of the body to be treated.

## Description

This invention relates to a running-water treatment device, particularly suitable for the anti-inflammatory treatment of body parts of human beings or animals.

It is well known that an inflamed articulation can be treated not only with medicaments but also by so-called "natural" treatment (to distinguish it from treatment with medicaments), consisting of cooling the articulation, for example by means of an ice pack or by immersing the articulation in cold or tepid water contained in a vessel. The ice has to be re-formed when dissolved or the water in the vessel replaced when warm as a result of contact with that part of the body concerned, and this has to be done fairly frequently.

The object of this invention is to provide a device which enables the relative part of the human or animal body to be treated in a simple and practical manner, so avoiding the use of medicaments.

This object is attained by the treatment device of the present invention, comprising a bag which is deformable to allow it to adapt to that part of the body to be treated, said bag being fed with cold or tepid water and being provided with a multiplicity of holes through which the water emerges, to wet with a continuous flow of water that part of the body to be treated.

As mains water or well water normally has a substantially constant temperature which is considerably less than body temperature, the bag can be fed directly with such water. However in those cases (in reality rather rare) in which the mains or well water has too high a temperature for the required purpose, the device can comprise a means for cooling the water to a suitable temperature, although this considerably complicates the device.

Conveniently the device comprises a means for maintaining the bag applied in the desired position to the body, notwithstanding any movements of the person or animal using the device. In particular said means can be an adjustable band fixed to said bag, to enable this latter to be maintained in the required position and cause it to adhere to that part of the body to be treated.

The effect obtained is basically that of a shower plus hydromassage, but with the advantage of being able to effect the treatment in any place in which a source of water is available and without the need for complicated and costly equipment, while at the same time achieving an optimized the application.

From the aforegoing it is apparent that the device of the invention is particularly useful for treating traumas, sprains, bursitis, desmitis, irritations and tendinitis.

It is also simple to use and reliably efficient, enabling the treated part to be decongested and the problem to be quickly solved at minimum cost.

Besides being able to treat the aforesaid affections, such a device can also be conveniently used in all those cases in which cooling of a part of the human or animal body is useful. Its availability is hence useful in those places in which sporting activities are carried out, and particularly for horses engaged in racing, trotting or galloping, for horses used for free-time activity, and for athletes.

The invention will be more apparent from the ensuing description of one embodiment thereof. In this description reference is made to the accompanying drawings, on which:
Figure 1 is a view of one side of the device of the invention in its extended state;
Figure 2 is a view thereof from the other side;
Figure 3 shows a first application in which the device is applied to a knee and to an elbow of a human being; and
Figure 4 shows its application to the four knees of a horse.

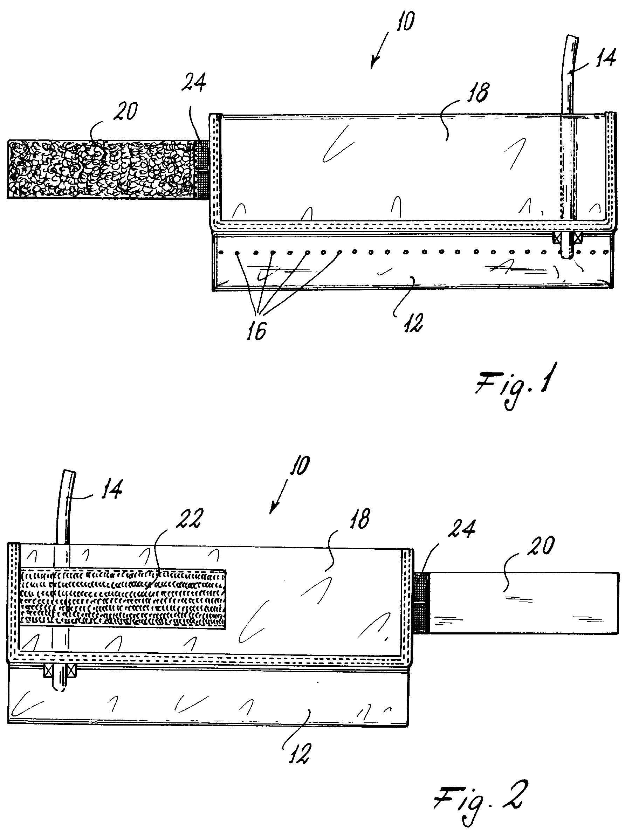

From Figures 1 and 2 it can be seen that the anti-inflammatory device 10 comprises an elongate impermeable bag 12 which can be fed with mains or well water via a flexible tube 14. For this purpose the free end of the tube 14 is connected to the water supply installation via a hose, not shown.

The bag 12 comprises in that side facing the observer (Figure 1) a series of small holes from which the water fed through the tube 14 can emerge.

To enable the bag 12 to be applied to the appropriate part of the human or animal body, the device 10 comprises a foldable support band 18, to the lower side of which there is fixed the upper edge of the bag 12. To one end of the band 18 there is fixed one end of a strap 20 having that face seen by the observer in Figure 1 covered with velcro, a region 22 (Figure 2) of the other face of the band 18 also being covered with velcro. The strap 20 is connected to the band 18 via an elasticized portion 24 which enables the device to better adapt to the relative part of the body.

The device 10 can be wrapped, for example, about a knee or an ankle and be fixed in position by the strap 20, which is made to engage the region 22 thereof, so locking it in position.

The support band 18 can be of a hemmed soft nylon fabric, whereas the bag 12 can be of sheet PVC and formed by bonding. The size of the holes 16 provided in one side of the bag 12 is such as to obtain a virtually constant flow therefrom determined by the water feed through the tube 14.

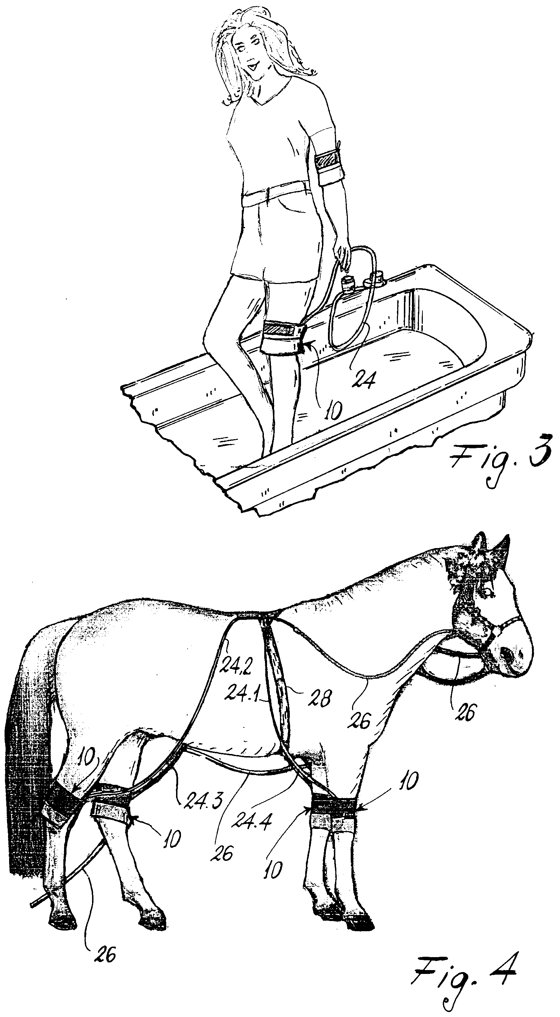

Figures 3 and 4 show two possible applications of the device 10. Specifically, Figure 3 shows the device 10 applied to the knee of a woman standing in a bath tub, the tube 14 (Figures 1 and 2) being connected by a hose 24 to the shower head connector normally provided on the tap assembly of bath tubs.

Figure 4 shows a system of hoses 24.1, 24.2, 24.3 and 24.4 connecting the tube 14 (Figures 1 and 2) of each device 10 to one end of a main hose 26, the other end of which is connected to the water main. The hose 26 preferably passes inside the ring to which the rope holding the horse is tied, then within the halter as far as the horse's back, where it terminates in the form of a threaded manifold (not shown) at a belt 28, for connection to a quick connector which enables the hose 26 to be connected to the secondary hoses 24.1, 24.2, 24.3 and 24.4.

As will be apparent from the aforegoing description, the advantages offered by the device of the invention are innumerable. In addition to the saving in the use of medicaments and the practicality of the device, using the device of the invention for an inflamed articulation results in a significantly high rate of shift of the treated human or animal body towards normality of its various parameters. Moreover, treatment with tepid water (the device then having to be fed by a source of tepid water or comprise a water heating device) accelerates healing and facilitates return to normal mechanical operability of a limb. Again, the hydromassage and the action of the cold water relax any contractures, increase hematic and lymphatic flow and alleviate pain, so facilitating the arrival of nutrient and reparative elements for the damaged area or facilitating the removal of excess liquids catabolites and free radicals.

In all cases the decongesting action of the device of the invention is exhibited immediately on initial application. The toning action provided by the normally cold but for certain treatments tepid water can be used both for treating limbs and for treating other parts of the human or animal body. In this latter case it may be necessary or convenient to provide suitable means (preferably using velcro) for holding the device in position. This latter can be fixed to the limbs such that the water runs onto the part to be treated, taking care that creases or improper positioning do not prevent optimum water outflow. To treat the aforelisted affections it is advisable to apply the device at least twice a day for a time of not less than twenty minutes but not greater than one hour (to prevent problems of hoof flaking in the case of horses), until the problem disappears.

The device is also useful for preventive purposes, to avoid tiring of the limbs and for muscle toning. For this purpose it must be used at the end of the working session for at least twenty minutes, or before the commencement of any heavier than usual work, to forestall any undesirable effects.

The device of the invention is an excellent means for defatiguing horses subjected to prolonged effort, or for retoning athletes resting between one competition and the next. In view of its practicality of use, the device can be easily used while travelling, wherever there is a water pipe available for its connection. The water to be fed to the device requires only a minimum pressure, sufficient for the water to run onto the relative body part. Too high a pressure is not only unnecessary but could also damage the device.

## Claims

1. A running-water treatment device for the human or animal body, comprising a bag which is deformable to allow it to adapt to that part of the body to be treated, said bag being fed with cold or tepid water and being provided with a multiplicity of holes through which the water emerges, to wet with a continuous flow of water that part of the body to be treated.

2. A treatment device as claimed in claim 1, wherein the water used is mains or well water.

3. A treatment device as claimed in claim 2, wherein if the mains or well water does not have a temperature suitable for the purpose, a means is provided to bring the water to the desired temperature.

4. A treatment device as claimed in claim 1, wherein a means is provided to maintain the bag applied in the required body position.

5. A treatment device as claimed in claim 4, wherein the means to maintain the bag in the required position comprises an adjustable and foldable band, one side of which is fixed to one side of the bag.

6. A treatment device as claimed in claim 5, particularly suitable for application to the knee or ankle, wherein from one end of the adjustable band there extends a strap, one face of which is covered with velcro and is engagable wholly or partly with a velcro-covered region provided on the other face of the adjustable band.

7. A treatment device as claimed in claim 6, wherein the strip is fixed to the band via an elasticized portion.
